(19) Europäisches Patentamt
European Patent Office
Office européen des brevets

(11) Veröffentlichungsnummer: **0 059 884**
**B1**

(12) # EUROPÄISCHE PATENTSCHRIFT

(45) Veröffentlichungstag der Patentschrift: **22.05.85**

(21) Anmeldenummer: **82101418.0**

(22) Anmeldetag: **25.02.82**

(51) Int. Cl.⁴: **C 07 D 307/79**, C 07 D 307/83, C 07 D 307/87, C 07 D 307/88, A 61 K 31/34, A 61 K 31/365

(54) **Neue Benzofuran-Derivate, ihre Herstellung und Verwendung.**

(30) Priorität: **11.03.81 DE 3110009**

(43) Veröffentlichungstag der Anmeldung: **15.09.82 Patentblatt 82/37**

(45) Bekanntmachung des Hinweises auf die Patenterteilung: **22.05.85 Patentblatt 85/21**

(84) Benannte Vertragsstaaten: **AT BE CH DE FR GB IT LI LU NL SE**

(56) Entgegenhaltungen:
**EP - A - 0 033 614**
**DE - A - 2 323 005**
**FR - A - 2 415 461**
**US - A - 3 947 587**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Schröder, Eberhard, Dr., verstorben (DE)**
Erfinder: **Lehmann, Manfred, Lutherstrasse 13, 1 Berlin 49 (DE)**
Erfinder: **Rufer, Clemens, Dr., Westhofener Weg 14, 1 Berlin 38 (DE)**
Erfinder: **Böttcher, Irmgard, Dr., Frobenstrasse 46, CH-4000 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Benzofuran-Derivate gemäss Patentanspruch 1, ein Verfahren zu ihrer Herstellung gemäss Patentanspruch 16 und pharmazeutische Präparate gemäss Patentanspruch 15, die diese Benzofuran-Derivate als Wirkstoff enthalten.

Die Substituenten $R_1$ und $R_2$ der Benzofuran-Derivate können gleichartig oder verschieden sein.

Die neuen Benzofuran-Derivate der allgemeinen Formel I können nach an sich bekannten Verfahren hergestellt werden. Ein geeignetes Herstellungsverfahren ist beispielsweise die im Anspruch 16 als Ausführungsform a) beschriebene Synthese.

Die Kondensation der Verbindungen der allgemeinen Formel II mit Methansulfonsäurechlorid oder Anhydrid gemäss dieser Verfahrensvariante erfolgt unter den bekannten Bedingungen, beispielsweise indem man die Sulfonsäurechloride in Gegenwart basischer Katalysatoren wie Natriumkarbonat, Natriumhydroxyd, Kaliumbikarbonat, Kaliumkarbonat, Pyridin, Lutidin oder Collidin mit den Verbindungen der allgemeinen Formel II umsetzt.

Die Cyclisierung der Phenoxyessigsäuren der allgemeinen Formel III gemäss Verfahrensvariante von Anspruch 16 zu den entsprechenden 3-Benzofuranonen gemäss Verfahrensvariante b) erfolgt beispielsweise mit starken, wasserabspaltend wirkenden Säuren, z.B. durch Erhitzen in Toluol mit p-Toluolsulfonsäure oder in Polyphosphorsäure oder durch Umsetzung z.B. mit Thionylchlorid oder Phosphorpentachlorid zu den Phenoxyessigsäurechloriden und deren Reaktion nach Friedel Crafts in Gegenwart entsprechender Katalysatoren wie Aluminiumchlorid etc.

Die erfindungsgemässen Verbindungen zeichnen sich durch eine gute antiphlogistische Wirksamkeit aus.

Darüberhinaus zeichnen sich die erfindungsgemässen Substanzen durch eine analgetische, antidysmenorrhöische, antipyretische, thrombocytenaggregationshemmende und diuretische Wirksamkeit aus. Darüberhinaus ist bemerkenswert, dass diese Substanzen die Prostaglandinsynthese kaum hemmen. Ein besonderer Vorzug dieser Verbindungen ist die grosse Dissoziation zwischen therapeutischer Wirksamkeit und unerwünschter Nebenwirkung (insbesondere Ulcerogenese).

Somit eignen sich die neuen Verbindungen in Kombination mit den in der galenischen Pharmazie üblichen Trägermitteln zur Behandlung von Erkrankungen des rheumatischen Formenkreises (wie der rheumatischen Arthritis, Ostearthritis oder ankylosierenden Spondalitis), Astha bronchiale, Heufiber u.a.

Bemerkenswert ist ferner, dass sich die Benzofuran-Derivate der allgemeinen Formel I gemäss Anspruch 1 darüberhinaus auch zur Behandlung von Migräne und von Dysmenorhoe eignen und das Thromboserisiko mindern.

Überraschenderweise gibt es unter den erfindungsgemässen Benzofuran-Derivaten auch solche, die zusätzlich noch eine ausgeprägte antiulcerogene sowie tumorhemmende Wirksamkeit besitzen.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die Ausgangsverbindungen für das erfindungsgemässe Verfahren gemäss Anspruch 17, Ausführungsform a) sind bekannt oder können in an sich bekannter Weise hergestellt werden.

So kann man beispielsweise die Verbindungen der allgemeinen Formel II durch Kondensation von Verbindungen der allgemeinen Formel IV mit Verbindungen der allgemeinen Formel V und anschliessende Reduktion der erhaltenen Nitroverbindungen der allgemeinen Formel VI herstellen. (In diesen Formeln haben $R_1$, $R_2$, X und –A–B– die obengenannte Bedeutung und Y bedeutet ein Chloratom, ein Bromatom oder ein Jodatom).

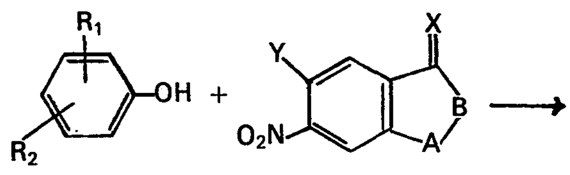

(IV), (V),

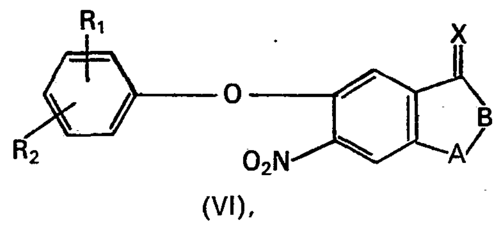

(VI),

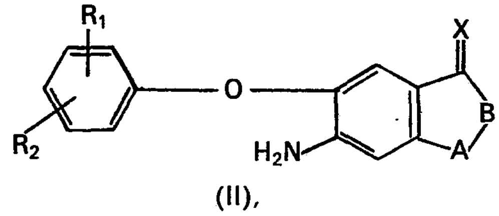

(II),

Die Verbindungen der allgemeinen Formel III kann man durch Kondensation der Verbindungen der allgemeinen Formel IV mit 4-Chlor-3-nitroanisol VII, Spaltung des Methylethers zur Verbindung der allgemeinen Formel IX, Kondensation mit Bromessigsäureethylester zur Verbindung der allgemeinen Formel X, Reduktion der Nitrogruppe zur Verbindung der allgemeinen Formel XI, Mesylierung der Aminogruppe zur Verbindung der allgemeinen Formel XII und Verseifung der Estergruppe herstellen:

(In diesen Formeln haben die Substituenten $R_1$ und $R_2$ ebenfalls die obengenannte Bedeutung).

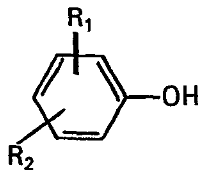

(IV),

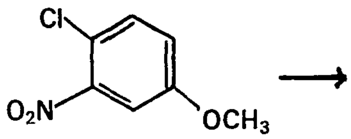

(VII),

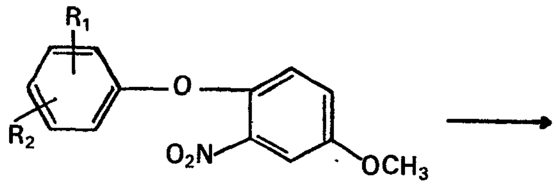

(VIII),

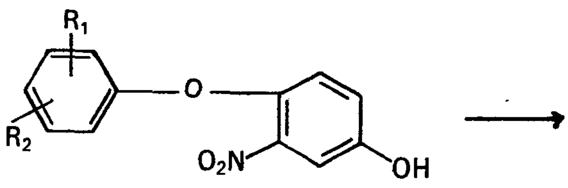

(IX),

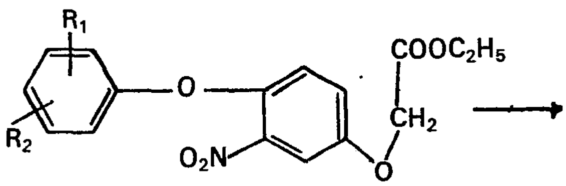

(X),

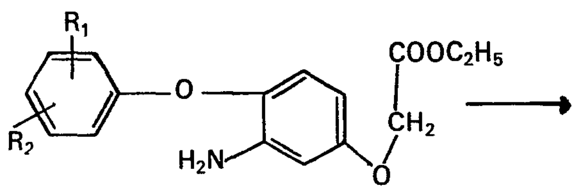

(XI),

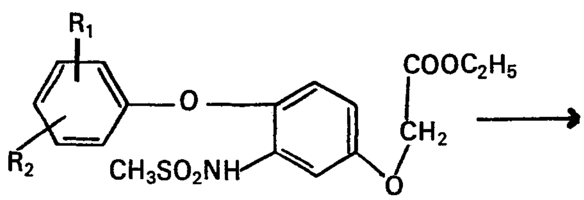

(XII),

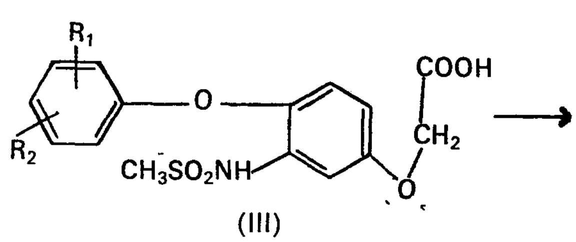

(III)

Die nachfolgenden Ausführungsbeispiele dienen zur Erläuterung des erfindungsgemässen Verfahrens.

Beispiel 1

a) 9,5 g 2,3-Dihydrobenzo[b]furan-5-amin in 40 ml Essigsäure und 10 ml Essigsäureanhydrid werden 1 1/2 Stunden auf 60 °C erwärmt und eingeengt.

Der Rückstand wird aus wässrigem Ethanol umkristallisiert.

Ausbeute: 11,5 g N-(2,3-Dihydrobenzo [b] furan-5-yl) -acetamid vom Schmelzpunkt 96 °C.

b) 440 g N-(2,3-Dihydrobenzo[b]furan-5-yl)acetamid in 4 l Essigsäure werden unter Eiskühlung in 30 Minuten mit 210 ml Salpetersäure (65%ig) versetzt und zwei Stunden nachgerührt.

Es wird mit Wasser verdünnt, das Kristallisat abgesaugt und mit Wasser gewaschen.

Ausbeute: 515 g N-(6-Nitro-2,3-dihydrobenzo [b] furan-5-yl) -acetamid vom Schmelzpunkt 141–142 °C.

c) 89 g N-(6-Nitro-2,3-dihydrobenzo [b] furan-5-yl)-acetamid in 1 Liter Ethanol und 400 ml Salzsäure werden zwei Stunden am Rückfluss gekocht und eingeengt. Der Rückstand wird mit 2 n Natronlauge/Essigsäureethylester versetzt, die wässrige Phase erneut einmal mit Essigsäureethylester ausgeschüttelt und die vereinigten organischen Phasen eingeengt. Aus Essigsäureethylester werden 66 g 6-Nitro-2,3-dihydrobenzo [b] furan-5-amin vom Schmelzpunkt 152 °C erhalten.

d) 54 g 6-Nitro-2,3-dihydrobenzo [b] furan-5-amin in 100 ml Essigsäure und 150 ml Wasser werden bei 5 °C mit 50 ml Schwefelsäure vereinigt, in 30 Minuten bei −5° bis 0 °C mit 21 g Natriumnitrit in 50 ml Wasser umgesetzt und 30 Minuten nachgerührt. (Lösung 1).

150 g Kupfer(II)sulfat und 93 g Natriumbromid werden in der Wärme in 800 ml Wasser gelöst und in 15 Minuten mit 39 g Natriumsulfit versetzt. Das ausgefallene Kupfer(I)bromid wird abgesaugt und in 750 ml 16%iger Bromwasserstoffsäure gelöst. (Lösung 2)

Lösung 1 wird in 15 Minuten bei 90 °C in Lösung 2 eingetropft (Badtemperatur 110 °C) und 10 Minuten bei 90 °C nachgerührt. Es wird abgekühlt, der Niederschlag abgesaugt, mit 2 n Salzsäure und Wasser gewaschen und aus Ethanol umkristallisiert.

Ausbeute: 68,5 g 5-Brom-6-nitro-2,3-dihydrobenzo [b]furan vom Schmelzpunkt 103 °C.

e) 2,95 g 5-Brom-6-nitro-2,3-dihydrobenzo [b] furan, 5,6 g Phenol, 5 g Kaliumcarbonat und 300 mg Kupfer(I)chlorid werden in 60 ml absolutem Pyridin zwei Stunden unter Stickstoff gekocht. Nach Einengen wurde der Rückstand in Essigsäureethylester/1 n Salzsäure aufgenommen, zweimal mit 1 n Salzsäure und dreimal mit 1 n Natronlauge ausgeschüttelt, die organische Phase getrocknet, eingeengt und zweimal aus Diisopropylether umkristallisiert.

Ausbeute: 2 g 6-Nitro-5-phenoxy-2,3-dihydrobenzo[b]furan vom Schmelzpunkt 94 bis 94,5 °C.

f) 2,3 g 6-Nitro-5-phenoxy-2,3-dihydrobenzo[b]furan in 90 ml Methanol werden 4 Stunden in Gegenwart von 2 g Raney Nickel GFE bei 70 bar hydriert, vom Katalysator abgesaugt, eingeengt und aus Methanol umkristallisiert.

Ausbeute: 1,7 g 5-Phenoxy-2,3-dihydrobenzo[b]furan -6-amin vom Schmelzpunkt 130 bis 131 °C.

g) 1,15 g 5-Phenoxy-2,3-dihydrobenzo [b] furan-6-amin in 10 ml Pyridin werden bei 0 °C mit 0,52 ml Methansulfonylchlorid versetzt. Nach drei Stunden bei 0 °C und 13 Stunden bei 20 °C wird eingeengt, der Rückstand in Chloroform aufgenommen, die Lösung dreimal mit 1 n Salzsäure gewaschen und eingeengt. Nach Umkristallisation des Rückstands aus Ethanol werden 1,2 g N-(5-Phenoxy-2,3-dihydrobenzo [b]furan-6-yl)- methansulfonamid vom Schmelzpunkt 133 °C erhalten.

Beispiel 2

a) 4,8 g 5-Brom-6-nitro-2,3-dihydrobenzo [b]furan, 3,4 g 2-Fluorphenol, 2 g Kupfer(I)-chlorid und 3,4 g Kalium-tert.-Butanolat werden in 70 ml tert.-Butanol fünfeinhalb Stunden gekocht. Es wurde eingeengt, der Rückstand in Essigsäureethylester/2 n Salzsäure aufgenommen, die organische Phase dreimal mit 2 n Salzsäure und dreimal mit 2 n Natronlauge gewaschen, eingeengt und der Rückstand über eine Kieselgel-Säule mit Toluol chromatographiert.

Es wurden 0,9 g 5-(2-Fluorphenoxy)-6-nitro-2,3-dihydrobenzo [b] furan als Öl und 0,9 g 5-(2-Fluorphenoxy)-6-nitro-benzo [b]furan vom Schmelzpunkt 91 °C erhalten (aus Diisopropylether).

b) 550 mg 5-(2-Fluorphenoxy)- 6-nitrobenzo [b]furan in 50 ml Methanol werden in 6 Stunden bei 70 bar in Gegenwart von 500 mg Raney-Nikkel GFE hydriert, filtriert und aus Ethanol umkristallisiert.

Ausbeute: 390 mg 5-(2-Fluorphenoxy)2,3-dihydrobenzo [b]furan-6-amin vom Schmelzpunkt 105–106,5 °C.

c) 370 mg 5-(2-Fluorphenoxy)-2,3- dihydrobenzo [b]furan-6-amin in 4 ml Pyridin werden wie im Beispiel 1 g) beschrieben mit 0,16 ml Methansulfonylchlorid umgesetzt.

Ausbeute: 395 mg N-[5-(2-Fluorphenoxy)-2,3-dihydrobenzo[b] furan-6-yl]-methansulfonamid vom Schmelzpunkt 111 bis 112 °C (aus Ethanol).

Beispiel 3

a) 24 g 5-Brom-6-nitro-2,3-dihydrobenzo[b]-furan, 20 g 2,4-Difluorphenol, 10 g Kupfer-(I)chlorid und 17 g Kalium-tert.-Butanolat in 350 ml tert.-Butanol werden 4 Stunden am Rückfluss gekocht, eingeengt und der Rückstand in Essigsäureethylester/2 n Salzsäure aufgenommen. Es wird dreimal mit 1 n Salzsäure und viermal mit 1 n Natronlauge ausgeschüttelt, die Essigsäureethylester-Phase eingeengt und das Rohprodukt auf einer Kieselgel-Säule (System: Toluol) gereinigt.

Ausbeute: 3,8 g 5-(2,4-Difluorphenoxy) -6-nitrobenzo [b]furan vom Schmelzpunkt 88–89 °C (aus Diisopropylether) und 15 g 5-(2,4-Difluorphenoxy)-6-nitro- 2,3-dihydrobenzo[b] furan als Öl.

b) Weg A

2,9 g 5-(2,4-Difluorphenoxy)-6-nitrobenzo [b]furan in 100 ml Methanol werden in Gegenwart von 3 g Raney-Nickel GFE in 6 Stunden bei 70 bar hydriert. Es wird vom Katalysator abgesaugt, eingeengt und aus Ethanol umkristallisiert.

Ausbeute: 2,4 g 5-(2,4-Difluorphenoxy)-2,3-dihydrobenzo[b] furan-6-amin vom Schmelzpunkt 74–75 °C.

Weg B

18 g 5-(2,4-Difluorphenoxy)-6- nitro-2,3-dihydrobenzo [b] furan in 500 ml Methanol werden wie in Beispiel 3 b) Weg A beschrieben hydriert und umkristallisiert.

Ausbeute: 5 g 5-(2,4-Difluorphenoxy)- 2,3-dihydrobenzo [b]furan-6-amin vom Schmelzpunkt 70–72,5 °C.

c) 2,1 g 5-(2,4-Difluorphenoxy) -2,3-dihydrobenzo[b] furan-6-amin, 0,8 ml Methansulfonylchlorid in 40 ml Pyridin werden 2 Stunden bei 0 °C und 14 Stunden bei Raumtemperatur gerührt und eingeengt. Das Rohprodukt wird in Chloroform gelöst, dreimal mit 1 n Salzsäure gewaschen, erneut eingeengt und zweimal aus Ethanol umkristallisiert.

Ausbeute: 2,55 g N-[5-(2,4-Difluorphenoxy)-2,3-dihydrobenzo [b]furan-6-yl]- methansulfonamid vom Schmelzpunkt 111,5 °C.

Beispiel 4

a) 3,6 g 5-Brom-6-nitro-2,3-dihydrobenzo [b]furan, 4,4 g 2-Chlor-4-fluorphenol, 3,3 g Kalium-tert.-Butanolat 1,5 g Kupfer(I)chlorid werden wie im Beispiel 2 a) beschrieben umgesetzt. Nach Reinigung auf einer Kieselgel-Säure (System: Toluol) werden 0,7 g 5-(2-Chlor-4-fluorphenoxy)- 6-nitrobenzo[b]furan vom Schmelzpunkt 103,5 bis 105 °C (aus Diisopropylether) und 2,7 g rohes 5-(2-Chlor-4-fluorphenoxy)6-nitro-2,3-dihydrobenzo[b] furan (nach mehrmaliger Kristallisation aus Methanol/Wasser Schmelzpunkt 86–88 °C) erhalten.

b) 2,7 g rohes 5-(2-Chlor-4-fluorphenoxy)- 6-nitro-2,3-dihydrobenzo[b] furan werden wie im Beispiel 1 f) beschrieben hydriert und aus wässrigem Ethanol umkristallisiert.

Ausbeute: 1,8 g 5-(2-Chlor-4-fluorphenoxy) -2,3-dihydrobenzo [b]furan-6-amin vom Schmelzpunkt 106–108 °C.

c) Analog Beispiel 1 g) werden aus 800 mg 5-(2-Chlor-4-fluorphenoxy)- 2,3-dihydrobenzo [b] furan-6-amin 800 mg N-[5-(2-Chlor-4-fluor-phenoxy)-2,3-dihydrobenzo [b]furan-6-yl] -methansulfonamid vom Schmelzpunkt 156–157 °C erhalten.

Beispiel 5

1,7 g N-[5-(2,4-Difluorphenoxy)- 2,3-dihydrobenzo [b]furan-6-yl]methansulfonamid in 15 ml Pyridin werden unter Eiskühlung mit

0,85 ml Essigsäureanhydrid versetzt, 3 Stunden bei 0 °C und 13 Stunden bei Raumtemperatur nachgerührt.

Es wird eingeengt, der Rückstand in Chloroform gelöst, dreimal mit 1 n Salzsäure gewaschen, eingeengt und zweimal aus Ethanol umkristallisiert.

Ausbeute: 1,65 g N-Acetyl-N- [5-(2,4-Difluorphenoxy) -2,3-dihydrobenzo [b] furan-6-yl]-methansulfonamid vom Schmelzpunkt 151,5–153 °C.

Beispiel 6

a) 37,5 g 4-Chlor-3-nitroanisol, 94 g Phenol, 5 g Kupfer(I)chlorid und 69 g Kaliumcarbonat in 400 ml Pyridin werden 6 Stunden am Rückfluss gekocht, eingeengt, der Rückstand in Essigsäureethylester/1 n Salzsäure aufgenommen, die organische Phase dreimal mit 1 n Salzsäure und dreimal mit 1 n Natronlauge gewaschen, getrocknet und eingeengt. Der Rückstand wird im Vakuum destilliert (Kochpunkt 0,053 mbar 143–146 °C) und aus Hexan umkristallisiert.

Ausbeute: 24,5 g (4-Methoxy-2-nitrophenyl)-phenylether vom Schmelzpunkt 33–34 °C.

b) 24,5 g (4-Methoxy-2-nitrophenyl)- phenylether in 150 ml Essigsäure, 100 ml Essigsäureanhydrid werden mit 200 ml Bromwasserstoffsäure (48%ig) versetzt und dreieinhalb Stunden gekocht und zum Teil eingeengt. Es wird mit Eiswasser versetzt und einmal mit Essigsäureethylester ausgeschüttelt. Die organische Phase wird viermal mit Wasser gewaschen und zweimal aus Diisopropylether umkristallisiert.

Ausbeute: 20 g 3-Nitro-4-phenoxyphenol vom Schmelzpunkt 101–102,5 °C.

c) 2,3 g 3-Nitro-4-phenoxyphenol in 20 ml absolutem Dimethylformamid werden bei 0 °C mit 360 mg Natriumhydrid (80%ig) 15 Minuten gerührt und mit 1,4 ml 2-Bromessigsäureethylester vereinigt. Nach drei Stunden bei Raumtemperatur wird eingeengt, der Rückstand in Essigsäureethylester/1 n Natronlauge aufgenommen und die organische Phase dreimal mit 1 n Natronlauge und zweimal mit Wasser gewaschen.

Ausbeute: 2,9 g 2-(3-Nitro-4-phenoxyphenoxy)-essigsäureethylester als Öl.

d) 10,5 g 2-(3-Nitro-4-phenoxyphenoxy)- essigsäureethylester in 500 ml Ethanol werden in Gegenwart von 10 g Raney-Nickel GFE in 4 Stunden bei 70 bar hydriert, filtriert, eingeengt und aus Ethanol umkristallisiert.

Ausbeute: 9 g 2-(3-Amino-4-phenoxyphenoxy)- essigsäureethylester vom Schmelzpunkt 87–88 °C.

e) 5,7 g 2-(3-Amino-4-phenoxyphenoxy)- essigsäureethylester in 50 ml Pyridin werden wie im Beispiel 1 g) beschrieben mit 2 ml Methansulfonylchlorid umgesetzt.

Ausbeute: 6,3 g 2-(3-Mesylamino-4-phenoxyphenoxy)- essigsäureethylester vom Schmelzpunkt 84 °C (aus Ethanol).

f) 5,8 g 2-(3-Mesylamino-4-phenoxyphenoxy)- essigsäureethylester in 160 ml Methanol und 24 ml 2 n Natronlauge werden zweieinhalb Stunden bei Raumtemperatur gerührt, zum Teil eingeengt, mit Eiswasser versetzt und mit 1 n Salzsäure angesäuert. Der Niederschlag wird in Essigsäureethylester gelöst, die organische Phase zweimal mit Wasser gewaschen, eingeengt und der Rückstand aus Essigsäureethylester/Hexan umkristallisiert.

Ausbeute: 5,2 g 2-(3-Mesylamino-4-phenoxyphenoxy)- essigsäure vom Schmelzpunkt 135–136 °C.

g) 3,75 g 2-(3-Mesylamino-4-phenoxyphenoxy)- essigsäure in 15 ml Thionylchlorid werden 5 Minuten gekocht und 30 Minuten bei Raumtemperatur nachgerührt. Es wird eingeengt, der Rückstand dreimal in 1,2-Dichlorethan gelöst und das Lösungsmittel wieder abdestilliert.

Das rohe Säurechlorid wird in 20 ml 1,2-Dichlorethan gelöst, in 30 Minuten bei −5 °C in 20 ml 1,2-Dichlorethan und 2,4 g Aluminiumchlorid eingetropft, 1 Stunde bei −5 °C nachgerührt und der Ansatz auf Eiswasser gegossen. Die organische Phase wird abgetrennt, getrocknet, eingeengt und der Rückstand auf einer Kieselgel-Säule (System: Cyclohexan/Essigsäureethylester 1,5+1) gereinigt.

Ausbeute: 1,08 g N-(3-Oxo-5-phenoxy-2,3-dihydrobenzo [b]furan-6-yl)- methansulfonamid vom Schmelzpunkt 163,5 bis 164,5 °C.

Beispiel 7

a) 46 g Kupfer(II)-sulfat · 5 $H_2O$ und 26,9 g Natriumbromid wurden in 160 ml Wasser bei 40 °C gelöst und 13,3 g Natriumsulfit wurden hinzugegeben (Entfärbung). Nach Kühlen auf 0 °C wurde abgesaugt, der Niederschlag in 160 ml Wasser aufgenommen und 13,2 g Natriumbromid sowie 66 ml 48%ige Bromwasserstoffsäure wurden hinzugefügt. Nach Erwärmen auf 70 °C entstand eine klare Lösung (Lösung 1). Zu einer Lösung von 10 g Natriumnitrit in 132 g konz. Schwefelsäure wurde bei 20–25 °C eine 30–40 °C warme Lösung von 23,5 g 2-Nitro-4,5-dimethylanilin in Essigsäure (470 ml) getropft (Lösung 2). Lösung 2 wurde nun langsam unter Rühren in die 70 °C warme Lösung 1 getropft. Nach 1 Stunde Nachrühren bei 70 °C wurde gekühlt, auf 2 l Eiswasser gegeben, abgesaugt und aus Ethanol umkristallisiert.

Ausbeute: 21,3 g 2-Brom-4,5-dimethyl-nitrobenzol vom Schmelzpunkt 60 °C.

b) 23 g 2-Brom-4,5-dimethyl-nitrobenzo, 22,4 g Kalium-tert.-butanolat, 20,6 g Phenol und 4 g Kupfer(I)chlorid wurden in 500 ml tert.-Butanol zweieinhalb Stunden gekocht. Nach Abkühlen und Einengen wurde mit 700 ml Äther versetzt und über Kieselgur filtriert. Das Filtrat wurde mit 1 n Natronlauge gewaschen, eingeengt und der Rückstand im Vakuum destilliert.

Ausbeute: 14,1 g (4,5-Dimethyl-2-nitro-phenoxybenzol vom Kochpunkt 0,23 mbar 156–157 °C.

c) 20 g 4,5-Dimethyl-2-nitro-phenoxybenzol in 80 ml Pyridin und 320 ml Wasser werden bei 80–90 °C in eineinhalb Stunden mit 207 g Kaliumpermanganat versetzt und 6 Stunden bei 90 °C

nachgerührt. Es wird mit 500 ml Wasser versetzt, abgesaugt, gut mit Wasser nachgewaschen und die Mutterlauge unter Eiskühlung mit 5 n Schwefelsäure angesäuert. Der Niederschlag wird abgesaugt, getrocknet, und aus Essigsäureethylester umkristallisiert.

Ausbeute: 13,9 g 4-Nitro-5-phenoxyphthalsäure vom Schmelzpunkt 164 °C.

d) 9 g 4-Nitro-5-phenoxyphthalsäure in 50 ml absolutem Tetrahydrofuran werden unter Stickstoff in 15 Minuten mit 450 ml einer 0,3 molaren Borantetrahydrofurankomplexlösung in Tetrahydrofuran versetzt und 16 Stunden bei Raumtemperatur nachgerührt. Unter Eiskühlung und unter Stickstoff wird vorsichtig bis zur Beendigung der Wasserstoffentwicklung mit Wasser versetzt, eingeengt und der Rückstand mit Essigsäureethylester verrieben. Es wird abgesaugt, die Mutterlauge eingeengt und auf einer Kieselgel-Säule (System: Dichlormethan/Aceton 1 + 1) gereinigt.

Ausbeute 5,5 g 4-Nitro-5-phenoxyphthalylalkohol vom Schmelzpunkt 85–86 °C Zersetzung (aus Ethanol/Wasser).

e) 2,75 g 4-Nitro-5-phenoxyphthalylalkohol in 60 ml absolutem Tetrahydrofuran werden mit 2,3 g p-Toluolsulfonylchlorid eine halbe Stunde bei Raumtemperatur gerührt, auf 0 °C abgekühlt und in 20 Minuten mit 720 mg Natriumhydrid (80%ig) versetzt. Es wird 1 Stunde bei 0 °C und 3 Stunden bei Raumtemperatur nachgerührt, eingeengt, der Rückstand in Essigsäureethylesther/Wasser gelöst, einmal mit 1 n Salzsäure und dreimal mit Wasser gewaschen und eingeengt. Das Rohprodukt wird auf einer Kieselgel-Säule (System: Cyclohexan/Essigsäureethylester 2 + 1) gereinigt.

Ausbeute: 1 g 5-Nitro-6-phenoxy-1,3-dihydroisobenzofuran vom Schmelzpunkt 77–78,5 °C (aus Ethanol).

f) 770 mg 5-Nitro-6-phenoxy-1,3-dihydrobenzofuran, 1 g Raney-Nickel GFE in 20 ml Ethanol werden in der Siedehitze in 5 Minuten mit 0,3 ml Hydrazinhydrat versetzt und 30 Minuten gekocht. Es wird abgesaugt, eingeengt und aus Ethanol umkristallisiert.

Ausbeute: 515 mg 6-Phenoxy-1,3-dihydroisobenzofuran-5-amin vom Schmelzpunkt 140–143,5 °C.

g) 477 mg 6-Phenoxy-1,3-dihydroisobenzofuran-5-amin in 5 ml Pyridin werden wie im Beispiel 1 g) beschrieben mit 0,24 ml Methansulfonylchlorid umgesetzt.

Ausbeute: 550 mg N-(6-Phenoxy-1,3-dihydroisobenzofuran-5-yl) -methansulfonamid vom Schmelzpunkt 159–160 °C.

Beispiel 8

a) 23 g 2-Brom-4,5-dimethyl-nitrobenzol und 28,6 g 2,4-Difluorphenol wurden wie in Beispiel 7 b) beschrieben umgesetzt. Ausbeute nach Destillation bei 150 °C Badtemperatur und 1,5 mmHg am Kugelrohr und Umkristallisation aus Benzin: 11,8 g 2-(2,4-Difluorphenoxy)-4,5-dimethyl-nitrobenzol vom Schmelzpunkt 82 °C.

b) 35 g 2-(2,4-Difluorphenoxy)-4,5-dimethylnitrobenzol in 190 ml Pyridin und 570 ml Wasser werden wie im Beispiel 7 c) beschrieben bei 80–90 °C mit 328 g Kaliumpermanganat oxidiert und aufgearbeitet.

Ausbeute: 31,8 g 5-(2,4-Difluorphenoxy)- 4-nitrophthalsäure vom Schmelzpunkt 152,5–155 °C (aus Ethanol/Wasser).

c) 3,36 g 5-(2,4-Difluorphenoxy)- 4-nitrophthalsäure in 20 ml absolutem Tetrahydrofuran werden wie im Beispiel 7 d) beschrieben mit 150 ml einer 0,3 molaren Borantetrahydrofurankomplexlösung in Tetrahydrofuran reduziert. Nach Kristallisation aus Ethanol/Wasser und Essigsäureethylester/Hexan werden 2,1 g 5-(2,4-Difluorphenoxy)- 4-nitrophthalylalkohol vom Schmelzpunkt 123,5–124,5 °C erhalten.

d) 2,8 g 5-(2,4-Difluorphenoxy)- 4-nitrophthalylalkohol werden mit 45 ml Bromwasserstoffsäure (48%ig) 1 Stunde bei 60 °C gerührt, mit Wasser versetzt, der Niederschlag abgesaugt und aus Ethanol umkristallisiert.

Ausbeute: 3,2 g 2-Brommethyl-5-(2,4-difluorphenoxy)- 4-nitro-benzylalkohol vom Schmelzpunkt 149–150 C.

e) 1,9 g 2-Brommethyl-5- (2,4-difluorphenoxy)4-nitro-benzylalkohol in 40 ml Dichlormethan werden mit 1,7 g Tetrabutylammoniumhydrogensulfat versetzt. Nach 5 Minuten mit 5 ml 1 n Natronlauge und nach 15 Minuten mit 10 ml 1 n Natronlauge vereinigt und 30 Minuten nachgerührt. Die Dichlormethan-Phase wird zweimal mit Wasser und zweimal mit 1 n Salzsäure gewaschen, getrocknet und eingeengt.

Der Rückstand wird auf einer Kieselgel-Säule (System: Cyclohexan/Essigsäureethylester 1 + 1) gereinigt und aus Diisopropylether umkristallisiert.

Ausbeute: 785 mg 6-(2,4-Difluorphenoxy)- 5-nitro-1,3-dihydroisobenzofuran vom Schmelzpunkt 105–106 °C.

f) 970 mg 6-(2,4-Difluorphenoxy)5-nitro-1,3-dihydroisobenzofuran in 80 ml Methanol werden in Gegenwart von 1 g Raney-Nickel GFE in 3 Stunden bei 20 bar hydriert, filtriert, eingeengt und aus Ethanol umkristallisiert.

Ausbeute: 770 mg 6-(2,4-Difluorphenoxy)-1,3-dihydroisobenzofuran-5-amin vom Schmelzpunkt 113,5–114,5 °C.

g) 736 mg 6-(2,4-Difluorphenoxy)- 1,3-dihydroisobenzofuran-5-amin in 8 ml Pyridin werden wie im Beispiel 1 g) mit 0,28 ml Methansulfonylchlorid umgesetzt.

Ausbeute: 750 mg N'[6-(2,4-Difluorphenoxy)-1,3-dihydroisobenzofuran-5-yl]- methansulfonamid vom Schmelzpunkt 176,5–178,5 °C.

Beispiel 9

a) 7,2 g 4-Nitro-5-phenoxyphthalsäure in 60 ml absolutem Tetrahydrofuran werden unter Stickstoff in 30 Minuten mit 160 ml einer 0,3 molaren Borantetrahydrofurankomplexlösung in Tetrahydrofuran versetzt und 16 Stunden bei Raumtemperatur nachgerührt. Unter Eiskühlung und unter Stickstoff wird mit Wasser versetzt, eingeengt, dreimal in Ethanol gelöst und das Lösungsmittel abdestilliert. Der Rückstand wird in Essigsäureethylester gelöst und mit 1,6 ml Schwefelsäure versetzt.

Nach 16 Stunden bei Raumtemperatur wird dreimal mit gesättigter Natriumhydrogencarbonat-Lösung gewaschen und die organische Phase eingeengt. Der Rückstand wird auf einer Kieselgel-Säule (System: Cyclohexan/Essigsäureethylester 1 + 1) gereinigt.

Ausbeute: 1,2 g 5-Nitro-6-phenoxyphthalid vom Schmelzpunkt 132–133 °C (aus Essigsäureethylester) und 1,2 g 6-Nitro-5-phenoxyphthalid vom Schmelzpunkt 166,5 bis 167,5 °C (aus Ethanol).

b) 950 mg 5-Nitro-6-phenoxyphthalid in 120 ml Methanol und 30 ml Tetrahydrofuran werden in Gegenwart von 2 g Raney-Nickel GFE in 3 Stunden bei 20 bar hydriert, filtriert und eingeengt.

Aus Methanol werden 570 mg 5-Amino-6-phenoxyphthalid vom Schmelzpunkt 205–206,5 °C erhalten.

c) 700 mg 5-Amino-6-phenoxyphthalid in 10 ml Pyridin werden wie im Beispiel 1 g) mit 0,3 ml Methansulfonylchloird umgesetzt.

Ausbeute: 740 mg N-(6-Phenoxyphthalid-5-yl)- methansulfonamid vom Schmelzpunkt 153–154 °C (aus Ethanol).

Beispiel 10

a) 4,6 g 5-(2,4-Difluorphenoxy)- 4-nitrophthalsäure in 20 ml absolutem Tetrahydrofuran werden wie im Beispiel 9 a) beschrieben mit 100 ml einer 0,3 molaren Borantetrahydrofurankomplexlösung in Tetrahydrofuran reduziert und aufgearbeitet.

Nach Kristallisation aus Ethanol werden 1,3 g 6-(2,4-Difluorphenoxy)- 5-nitrophthalid vom Schmelzpunkt 164 bis 165,5 °C erhalten.

b) 1,2 g 6-(2,4-Difluorphenoxy)-5- nitrophthalid in 120 ml Methanol und 30 ml Tetrahydrofuran werden wie im Beispiel 9 b) beschrieben reduziert.

Ausbeute: 1 g 5-Amino-6- (2,4-difluorphenoxy)-phthalid vom Schmelzpunkt 96–98 °C.

c) 970 mg 5-Amino-6- (2,4-difluorphenoxy)-phthalid in 10 ml Pyridin werden wie im Beispiel 1 g) beschrieben mit 0,32 ml Methansulfonylchlorid umgesetzt.

Ausbeute: 930 mg N-[6-(2,4-Difluorphenoxy)-phthalid-5-yl]- methansulfonamid vom Schmelzpunkt 155–156,5 °C.

**Patentansprüche**

1. Benzofuran-Derivat der allgemeinen Formel I

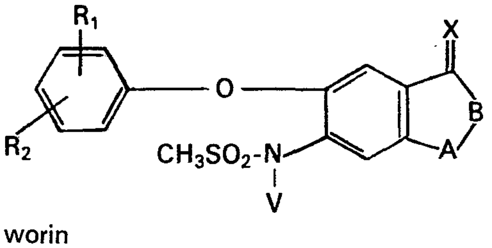
(I),

worin

V ein Wasserstoffatom oder eine Acetylgruppe und

X eine Oxogruppe oder zwei Wasserstoffatome darstellen, und worin

$R_1$ und $R_2$ gleich oder verschieden sind und Wasserstoffatome, Fluoratome oder Chloratome darstellen und –A–B– die Gruppierungen $-O-CH_2-$ oder $-CH_2-O-$ bedeuten.

2. Benzofuran-Derivate gemäss Patentanspruch 1, dadurch gekennzeichnet, dass V ein Wasserstoffatom bedeutet.

3. Benzofuran-Derivate gemäss Patentanspruch 1, dadurch gekennzeichnet, dass $R_1$ ein orthoständiges Fluoratom und $R_2$ ein paraständiges Fluoratom darstellen.

4. Benzofuran-Derivate gemäss Patentanspruch 1 bis 3, dadurch gekennzeichnet, dass –A–B– die Gruppierung $-CH_2O-$ bedeutet.

5. N-(5-Phenoxy-2,3-dihydrobenzo [b] furan-6-yl)- methansulfonamid.

6. N- (5-(2-Fluorphenoxy)- 2,3-dihydrobenzo [b] furan-6-yl]- methansulfonamid.

7- N- [5-(2,4-Difluorphenoxy)- 2,3-dihydrobenzo[b] furan-6-yl]- methansulfonamid.

8. N- [5-(2-Chlor-4-fluor-phenoxy)- 2,3-dihydrobenzo[b] furan-6-yl] methansulfonamid.

9. N-Acetyl-N- [5-(2,4-difluorphenoxy) -2,3-dihydrobenzo [b] furan-6-yl] -methansulfonamid.

10. N-(3-Oxo-5-phenoxy-2,3-dihydrobenzo-[b] furan-6-yl)- methansulfonamid.

11. N-(6-Phenoxy-1,3-dihydroisobenzofuran-5-yl) -methansulfonamid.

12. N- [6-(2,4-Difluorphenoxy)- 1,3-dihydroisobenzofuran-5-yl]- methansulfonamid.

13. N- (6-Phenoxyphthalid-5- yl)-methansulfonamid.

14. N-[6- (2,4-Difluorphenoxy)- phthalid-6-yl]-methansulfonamid.

15. Pharmazeutische Präparate, gekennzeichnet durch ein oder zwei Benzofuran-Derivate gemäss Anspruch 1 bis 14 als Wirkstoff.

16. Verfahren zur Herstellung von Benzofuran-Derivaten gemäss Anspruch 1, dadurch gekennzeichnet, dass man

a) eine Verbindung der allgemeinen Formel II

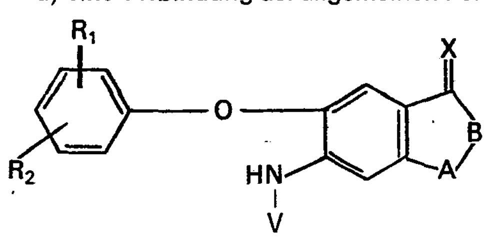
(II),

worin

$R_1$, $R_2$, X, V und –A–B– die im Anspruch 1 genannte Bedeutung besitzen, mit Methansulfonsäure-chlorid oder -anhydrid kondensiert, und gegebenenfalls die Benzofuran-Derivate der allgemeinen Formel I mit V in der Bedeutung von Wasserstoff acetyliert, oder

b) zur Herstellung von Benzofuran-Derivaten gemäss Anspruch 1 mit –A–B– in der Bedeutung einer $-O-CH_2$-Gruppe und X in der Bedeutung einer Oxogruppe eine Verbindung der allgemeinen Formel III

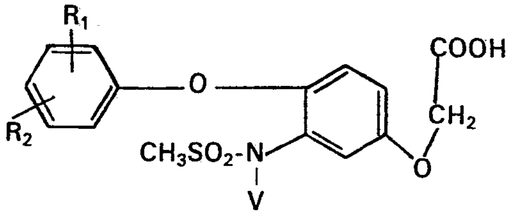
(III),

worin

$R_1$, $R_2$ und V die obengenannte Bedeutung besitzen, cyclisiert und gegebenenfalls Benzofuran-Derivate mit V in der Bedeutung eines Wasserstoffatoms acetyliert.

**Revendications**

1. Dérivés du benzofuranne répondant à la formule générale I:

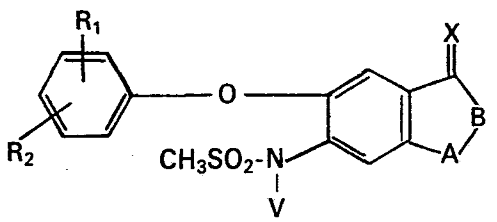
(I),

dans laquelle

V représente un atome d'hydrogène ou un radical acétyle

X représente un radical oxo ou 2 atomes d'hydrogène,

$R_1$ et $R_2$, qui peuvent être identiques l'un à l'autre ou différents l'un de l'autre, représentent chacun un atome d'hydrogène, un atome de fluor ou un atome de chlore, et

–A–B– représente l'un des groupements $-O-CH_2-$ et $-CH_2-O-$.

2. Dérivés du benzofuranne selon la revendication 1, caractérisés en ce que V représente un atome d'hydrogène.

3. Dérivés du benzofuranne selon la revendication 1, caractérisés en ce que $R_1$ représente un atome de fluor en position ortho et $R_2$ un atome de fluor en position para.

4. Dérivés du benzofuranne selon l'une quelconque des revendications 1 à 3, caractérisés en ce que –A–B– représente le groupement $-CH_2-O-$.

5. N-(Phénoxy-5 dihydro-2,3 benzo[b]furannyl-6-)-méthane-sulfonamide.

6. N-[(Fluoro-2 phénoxy)-5 dihydro-2,3 benzo[b]furannyl-6]-méthane-sulfonamide.

7. N-[(Difluoro-2,4 phénoxy)-5 dihydro-2,3 benzo[b]furannyl-6]-méthane-sulfonamide.

8. N-[(Chloro-2- fluoro-4 phénoxy)-5- dihydro-2,3 benzo[b] furannyl-6]- méthane-sulfonamide.

9. N-Acéthyl-N-[(difluoro-2,4 phénoxy)-5 dihydro-2,3 benzo[b]furannyl-6] méthane-sulfonamide.

10. N-(Oxo-3 phénoxy-5 dihydroxy-2,3 benzo[b] furannyl-6)-méthane-sulfonamide.

11. N-(Phénoxy-6 dihydro-1,3 isobenzofurannyl-5) -méthane-sulfonamide.

12. N-[(Difluoro-2,4 phénoxy)-6 dihydro-1,3 isobenzofurannyl-5]-méthane-sulfonamide.

13. N-(Phénoxy-6 phtalidyl-5)-méthane-sulfonamide.

14. N-[(Difluoro-2,4 phénoxy)-6 phtalidyl-5]-méthane-sulfonamide.

15. Compositions pharmaceutiques caractérisées en ce qu'elles contiennent, comme substance active, un ou deux dérivés du benzofuranne selon l'une quelconque des revendications 1 à 14.

16. Procédé de préparation de dérivés du benzofuranne selon la revendication 1, procédé caractérisé en ce que:

a) on condense un composé répondant à la formule générale II:

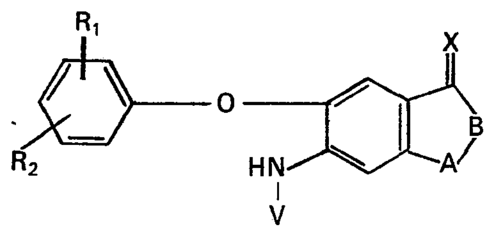
(II),

dans laquelle $R_1$, $R_2$, X, V et –A–B– ont les significations données à la revendication 1, avec le chlorure ou l'anhydride de l'acide méthane-sulfonique, et, le cas échéant, on acétyle les dérivés du benzofuranne de formule générale I dans lesquels V représente l'hydrogène, ou

b) pour préparer les dérivés du benzofuranne selon la revendication 1 dans lesquels –A–B– représente un radical $-O-CH_2-$ et X représente un radical oxo on acétyle un composé répondant à la formule générale III:

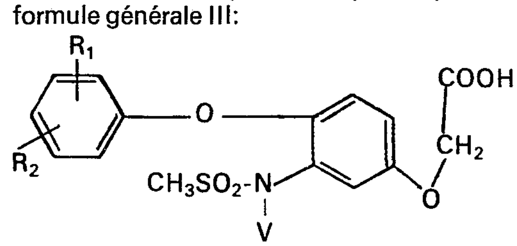
(III)

dans laquelle $R_1$, $R_2$ et V ont les significations précédemment données, et le cas échéant, on acétyle les dérivés du benzofuranne dans lesquels V représente un atome d'hydrogène.

**Claims**

1. Benzofuran derivatives of the general formula I

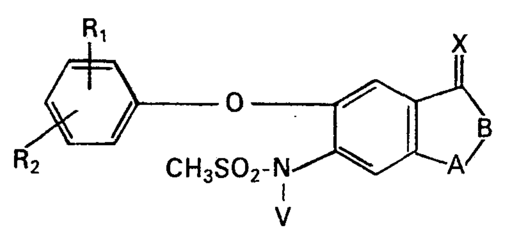
(I),

in which

V represents a hydrogen atom or an cetyl group and

X represents an oxo group or two hydrogen atoms, and in which

$R_1$ and $R_2$ are identical or different and represent hydrogen atoms, fluorine atoms or chlorine atoms and

–A–B– represents the grouping $-O-CH_2-$ or $-CH_2-O-$.

2. Benzofuran derivatives according to patent claim 1, characterised in that V represents a hydrogen atom.

3. Benzofuran derivatives according to patent claim 1, characterised in that $R_1$ represents a fluorine atom in the ortho-position and $R_2$ represents a fluorine atom in the para-positon.

4. Benzofuran derivatives according to patent claims 1 to 3, characterised in that –A–B– represents the grouping $-CH_2O-$.

5. N-(5-phenoxy-2,3-dihydrobenzo[b]furan-6-yl)-methanesulphonamide.

6. N-[5-(2-fluorophenoxy)-2,3-dihydrobenzo[b]furan-6-yl]-methanesulphonamide.

7. N-[5-(2,4-difluorophenoxy)-2,3-dihydrobenzo[b]-furan-6-yl]-methanesulphonamide.

8. N-[5-(2-chloro-4-fluorophenoxy)-2,3-dihydrobenzo [b] furan-6-yl]-methanesulphonamide.

9. N-acetyl-N-[5-(2,4-difluorphenoxy)-2,3-dihydrobenzo [b] furan-6-yl] -methanesulphonamide.

10. N-(3-oxo-5-phenoxy-2,3-dihydrobenzo [b] furan-6-yl)methanesulphonamide.

11. N-(6-phenoxy-1,3-dihydroisobenzofuran--5-yl)-methanesulphonamide.

12. N-[6-(2,4-difluorophenoxy)-1,3-dihydroisobenzofuran-5-yl]-methanesulphonamide.

13. N-(6-phenoxyphthalid-5-yl)-methanesulphonamide.

14. N-[6-(2,4-difluorophenoxy)-phthalid-5-yl]-methanesulphonamide.

15. Pharmaceutical preparations characterised by one or two benzofuran derivatives according to claims 1 to 14 as active ingredient.

16. Process for the manufacture of benzofuran derivatives according to claim 1, characterised in that

a) a compound of the general formula II

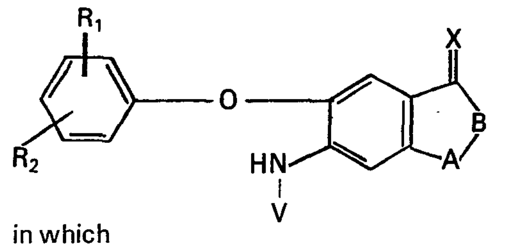
(II),

in which

$R_1$, $R_2$, X, V and –A–B– have the meanings given in claim 1, is condensed with methanesulphonic acid chloride or anhydride and, optionally, the benzofuran derivatives of the general formula I in which V represents hydrogen are acetylated, or

b) for the manufacture of benzofuran derivatives according to claim 1 in which –A–B– represents a $-O-CH_2-$ group and X represents an oxo group, a compound of the general formula III

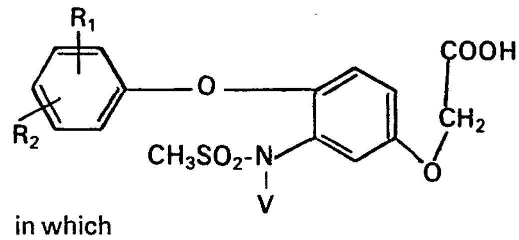
(III)

in which

$R_1$, $R_2$ and V have the meanings given above, is cyclised and, optionally, benzofuran derivatives in which V represents a hydrogen atom are acetylated.